# FASCICULE DE BREVET EUROPEEN

(11) **EP 0 318 338 B1**
(45) Date de publication et mention de la délivrance du brevet: **08.12.1993**
(21) Numéro de dépôt: 88401051.3
(22) Date de dépôt: 29.04.1988
(51) Int. Cl.: C07K 1/06

(54) **Procédé de préparation de synthons peptidiques**
Verfahren zur Herstellung von Peptidsynthonen
Process for the preparation of peptide synthons

(30) Priorité: 25.11.1987 FR 8716341
(43) Date de publication de la demande: 31.05.1989
(73) Titulaire: RHONE-POULENC CHIMIE, 92408 Courbevoie Cédex (FR)
(72) Inventeur: Jacquier, Robert, F-34100 Montpellier (FR); Verducci, Jean, F-34670 Baillargues (FR); Ibea, Michel, F-34000 Montpellier (FR)
(74) Mandataire: Le Pennec, Magali

(56) Documents cités:
- CHEMISCHE BERICHTE, vol. 94, no. 5, 18 mai 1961, pages 1263-1267, Verlag Chemie GmbH, Weinheim/Bergstr., DE; L. BIRKOFER et al.: "Di- und Tripeptide aus silylierten Aminosäuren; der Trimethylsilylrest als Schutzgruppe für SH- und OH-Funktionen"
- CHEMICAL ABSTRACTS, vol. 105, no. 3, 21 juin 1986, page 676, résumé no. 24609t, Columbus, Ohio, US; W. KEESE et al.: "2-Chloro-1-methylpyridinium iodide: a suitable coupling reagent in peptide synthesis", & BIOL. CHEM. HOPPE-SEYLER 1985, 366(12), 1093-5
- CHEMICAL ABSTRACTS, vol. 100, no. 15, 9 avril 1984, page 641, résumé no. 121575h, Columbus, Ohio, US; K. TAKEDA et al.: "Studies on activating methods of functional groups. Part IX. A convenient synthesis of peptide using oxdates", & TETRAHEDRON LETT. 1983, 24(41), 4451-4
- TETRAHEDRON LETTERS, no. 39, septembre 1971, pages 3595-3598, Pergamon Press, Oxford, GB; S.I. YAMADA et al.: "A new method for the synthesis of peptides using the adducts of phosphorus compounds and tetrahalomethanes"
- CHEMICAL ABSTRACTS, vol. 89, no. 13, 25 septembre 1978, page 1001, résumé no. 116357a, Columbus, Ohio, US; H.D. JAKUBKE et al.: "Lewis acids, especially zinc chloride: a new type of carbodimide additive in peptide synthesis", & TETRAHEDRON LETT. 1978, (17), 1497-500
- CHEMICAL ABSTRACTS, vol. 98, no. 13, 28 mars 1983, page 643, résumé no. 107745x, Columbus, Ohio, US; E. BERGER et al.: "Studies on racemization in peptide synthesis using the mixed anhybride method", && Z. CHEM. 1982, 22(10), 379-80
- CHEMICAL ABSTRACTS, vol. 85, no. 19, 8 novembre 1976, page 588, résumé no. 193089x, Columbus, Ohio, US; E.K. STEPURENKO et al.: "Use of tris(alpha-pyridyl) phosphite in peptide synthesis", & TEZISY DOKL. VSES. SIMP. KHIM. PEPT. BELKOV. 3rd 1974, 141
- CHEMICAL ABSTRACTS, vol. 107, no. 1, 6 juillet 1987, page 707, résumé no. 7620p, Columbus, Ohio, US; & JP-A-62 59 295 (SUNTORY LTD) 14-03-1987

## Description

La présente invention concerne un procédé de préparation de synthons peptidiques. Elle concerne plus particulièrement une nouvelle méthode de synthèse peptidique non racémisante.

Il est connu d'après certains ouvrages spécialisés tels que par exemple (The Peptides, Vol. 1, Academic Press, 1979 ou Principles of peptides synthesis, Springer, 1984) de réaliser des synthèses peptidiques par condensation d'une chaîne peptidique dont la terminaison acide est activée (E) et la terminaison amine est protégée (P) avec une autre chaîne peptidique dont seule la terminaison acide est estérifiée en présence d'une base organique qui permet de neutraliser le groupe partant EOH qui est dans la majorité des cas acide selon les réactions suivantes :

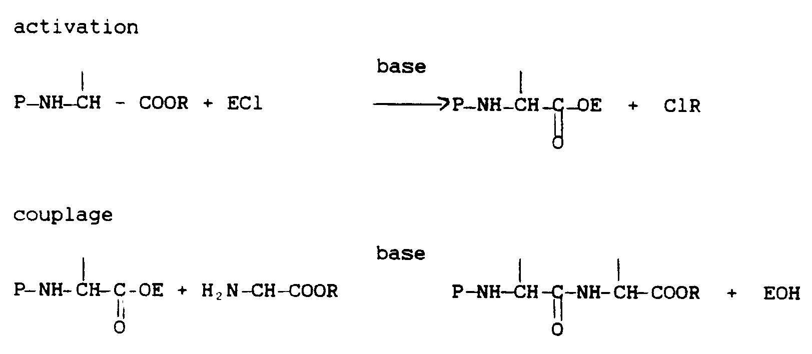

La base utilisée quelle qu'elle soit provoque une racémisation important du motif peptidique soit au cours de l'étape d'activation soit au cours de l'étape de couplage.

Or, il est bien connu en synthèse peptidique que la plupart des peptides ne sont actifs que sous une seule forme diastéréoisomère. La racémisation provoque donc une perte d'activité des produits obtenus après la condensation, ce qui est très nuisible car les matières premières utilisées chiralement actives sont d'un prix souvent très élevé. Les peptides étant utilisés dans l'industrie pharmaceutique qui exige des normes analytiques sévères doivent être purifiés s'ils sont obtenus sous forme de mélange de diastéréoisomères lors de leur synthèse. Cette purification est très onéreuse.

L'industrie est donc à la recherche depuis longtemps de procédés chimiques concurrentiels des procédés d'extraction à partir de produits naturels qui permettent d'atteindre des peptides actifs d'une pureté chirale bien définie et à un coût pouvant concurrencer les procédés d'extraction.

Il est connu par exemple de préparer les peptides en inhibant au maximum la racémisation par l'utilisation d'agents activants tels que le dicyclohexylcarbodiimide (DCC) et d'additifs tels que :
le N-hydroxysuccinimide,
l'hydroxy-1 benzotriazole,
le N-hydroxy norbornène-5 dicarboximide-2,3.

Il est généralement connu de condenser sans trop de racémisation des aminoacides N-protégés par des groupes uréthannes par une technique de synthèse permettant d'additionner à un peptide les acides aminés un par un. Par contre, lorsque la fonction amine est substituée par un groupe acyle (Miyazawa, Yamada et Kuwata, Peptide Chemistry, 1982, 69) ou fait partie d'une chaîne peptidique, le taux de racémisation n'est plus négligeable et peut atteindre 25 %.

Le taux de racémisation varie ainsi avec l'acide aminé, le groupe protecteur, le réactif activant, les conditions de la réaction d'activation et en particulier peut être total lorsque l'on utilise un groupe protecteur du type acyle.

La présente invention permet de résoudre les problèmes laissés dans l'art antérieur. Elle permet par une voie chimique à partir de matières premières peu onéreuses d'accéder à des synthons peptidiques présentant une pureté chirale égale ou supérieure à 99 % lors du couplage par exemple de la valine sur une autre valine, qui avec d'autres conditions expérimentales (activation du type : DCC et additif ou chlorure de pivaloyle et amine tertiaire ou BPO Cl et amine tertiaire) conduit à une racémisation non négligeable.

Elle a pour objet un procédé de préparation de synthons peptidiques optiquement actifs caractérisé en ce que :
- dans une première étape on prépare un dérivé O-silylé (I) d'un peptide ou d'un aminoacide dont la fonction axotée est protégée,
- dans une deuxième étape on active le peptide ou l'aminoacide O-silylé par un sel complexe (c'est-à-dire un sel comportant un anion complexe de formule MX⁻ₙ₊₁) de chloroimmonium ou de dichlorophosphénium,
- dans une troisième étape on condense le peptide ou l'aminoacide activé (III) sur un peptide ou un aminoacide dont la fonction amine a été N-sylilée.

La présente invention exclut donc toute intervention de composés basiques particulièrement dans l'étape d'activation (B) et dans l'étape de couplage (C). Elle est particulièrement intéressante lorsque l'on part d'un peptide O-silylé contenant plus d'un aminoacide.

Il est aussi connu d'après le brevet européen n° 184243 de préparer des dérivés silylés d'acides aminés ou de peptide à l'aide de trialkylcyanosilanes puis à coupler ces dérivés silylés avec des aminoacides ou des peptides activés. Lors de la silylation il y a libération d'acide cyanhydrique dont la toxicité est telle qu'elle a fait exclure ce procédé de tous les programmes de l'industrie. D'autre part les agents d'activation utilisés dans ce brevet ne sont pas utilisables sur des fragments peptidiques sans apparition d'une racémisation importante.

L'ensemble de la réaction de la présente invention peut être schématisé par les équations suivantes : 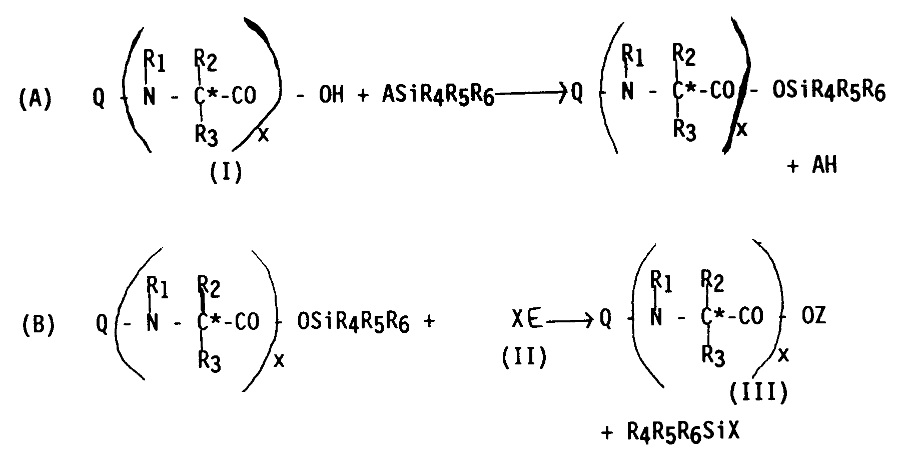 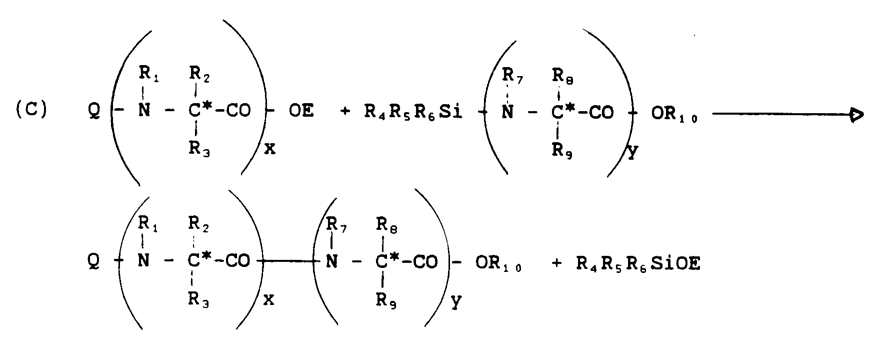
dans lesquelles :
. A représente le chlore ou un groupe 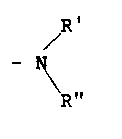 dans lequel :
   * R′ représente l'hydrogène, un groupe alkyle CₙH_{2n + 1} avec n compris entre 1 et 4,
   * R˝ représente un groupe alkyle CₙH_{2n + 1} avec n compris entre 1 et 4 ou un groupe trialkylsilyl dans lequel le groupe alkyl contient 1 à 4 atomes de carbone,
   * R′ et R˝ peuvent aussi former un groupe alkylsilyloxyalkylidène et notamment le groupe

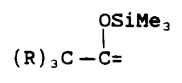

   - R représentant un hydrogène ou un fluor.
. Q est un des groupes protecteurs de la fonction amine N-terminale utilisés dans les méthodes connues dans l'art antérieur (Gross et Meienhofer, The peptides, Vol. 3, Academic Press, 1981). A titre d'exemples non limitatifs, Q peut être le groupe t-butyloxycarbonyle (Boc), benzyloxycarbonyle (Z), fluorénylméthyloxycarbonyle (Fmoc), benzoyle, trifluoroacétyle, formyle ;
. R₁ et R₇ sont soit un hydrogène, soit un groupe méthyle
. R₂ et R₈ peuvent être choisis parmi les substituants suivants l'hydrogène, les groupes alkényles ou alkyles CₙH_{2n + 1} linéaires ou ramifiés, n possédant des valeurs entières comprises entre 1 et 4, un groupe benzyle ou l'un des groupes dont la liste non limitative figure ci-après : 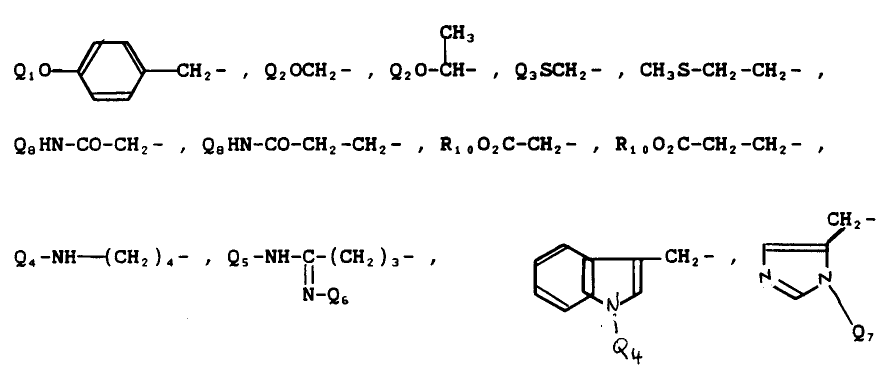 dans lesquels Q₁, Q₂, Q₃, Q₄, Q₅, Q₆, Q₇ et Q₈ sont des groupes protecteurs des chaînes latérales utilisés dans les méthodes connues dans l'art antérieur (Gross et Meienhofer, The peptides, Vol. 3, Academic Press, 1981). A titre d'exemples non limitatifs, Q₁ peut être le groupe benzyle, bromo-2 benzyle ou dichloro-2,6 benzyle, Q₂ le groupe benzyle ou t-butyle, Q₃ le groupe benzyle, t-butyle, trityle, acétamidométhyle ou benzamidométhyle, Q₄ le groupe trifluoroacétyle, t-butyloxycarbonyle ou benzyloxycarbonyle, Q₅ peut être le groupe nitro, p-méthoxybenzènesulfonyle ou mésitylènesulfonyle lorsque Q₆ = H, ou bien Q₅ et Q₆ peuvent être simultanément constitués par le groupe adamantyloxycarbonyle, Q₇ peut être le groupe phénacyle, benzyloxyméthyle ou t-butoxyméthyle, Q₈ peut être enfin le groupe benzhydryle, dimethoxybenzydryle ou xanthydryle.
. R₃ et R₉ sont choisis parmi les substituants : hydrogène, ou un groupe alkyle CₙH_{2n + 1} avec n compris entre 1 et 4.
. R₁ avec R₂ ou R₇ avec R₈ peuvent aussi former une chaîne cyclopolyméthylénique contenant 2 à 5 atomes de carbone, les atomes de carbone sur lesquels sont fixés les substitutions R₂ et R₈ possèdent la configuration L ou D, ils sont asymétriques (*) sauf si R₂ = R₃ et R₈ = R₉
. x et y sont des nombres entiers compris entre 1 et 15
. R₄, R₅ et R₆ sont choisis parmi l'hydrogène, les groupes alkyles CₙH_{2n + 1}, avec n compris entre 1 et 4, à condition que ces trois termes ne soient pas simultanément des hydrogènes,
. R₁₀ est un des groupes protecteurs de la fonction acide C-terminale et des fonctions acides des chaînes latérales utilisés dans les méthodes connues dans l'art antérieur (Gross et Meienhofer, The peptides, Vol. 3, Academic Press, 1981). A titre d'exemples non limitatifs, on peut citer les groupes méthyle, éthyle, phényle benzyle ou t-butyle.
. XE représente le réactif d'activation.

Le réactif d'activation XE est choisi parmi les sels complexes de chloroimmonium les sels complexes d'halogénure ou d'oxyhalogénure de phosphore et les sels complexes d'halogénure d'oxalyle.

Les sels complexes de chloroimmonium répondent à la formule générale (IV) : 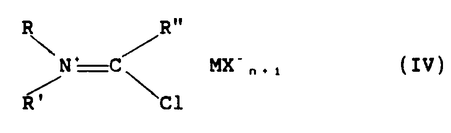
dans laquelle :
* X représente un halogène choisi parmi le chlore, le brome et l'iode.
* R et R′, identiques ou différents, représentent un radical alkyle ayant de préférence de 1 à 5 atomes de carbone, ou encore un radical divalent de formule

   -(CH₂)ₘ-(Y)_{m′} - (CH₂)ₘ-

   dans laquelle :
   . Y est un groupe méthylène ou un hétéroatome tel que l'atome d'oxygène ou d'azote,
   . m est un nombre entier de 2 à 6, de préférence égal à 2,
   . m′ est un nombre entier égal à 0 ou 1,
* R'' est un atome d'hydrogène ou un groupement alkyle, alkényle, aryle ou aralkyle.
* M représente un métal choisi parmi le zinc, l'aluminium, l'antimoine, le titane et l'étain.
* n est un nombre entier égal ou supérieur à 2 et égal ou inférieur à 5.

On préfère utiliser parmi les métaux cités le zinc et l'antimoine V.

Les sels complexes d'halogénure de phosphore répondent à la formule générale (V), ce sont des produits nouveaux non décrits dans l'art antérieur 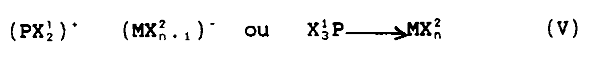
dans laquelle
. M représente l'antimoine et n = 5, ou le zinc et n = 2
. X¹ et X² représentent un halogène identique ou différent choisi parmi le chlore, le brome ou l'iode.

Les sels complexes d'halogénure d'oxalyle sont préparés in situ et ne sont pas isolés.

Le procédé de préparation des complexes de formule (IV) est réalisé par addition d'halogénure métallique à un sel de chloroimmonioum et pour les autres complexes par addition d'halogénure d'antimoine V ou de zinc à l'halogénure de phosphore ou à l'halogénure d'oxalyle. Le procédé de préparation est réalisé de préférence à une température inférieure à 0°C.

Ces complexes sont préparés dans les solvants choisis parmi les solvants aliphatiques chlorés et de préférence parmi le chlorure de méthylène et le chloroforme.

La réaction de silylation de la fonction acide est de préférence réalisée à partir d'un aminoacide ou d'un peptide N-protégé, par réaction avec un réactif silylant de formule ASiR₄R₅R₆ dans laquelle les termes A,R₄,R₅ et R₆ ont la même signification que précédemment.

La réaction de silylation de la fonction amine est de préférence réalisée à partir d'un aminoacide ou d'un peptide par réaction entre un ester correspondant et un réactif silylant de formule ASiR₄R₅R₆ dans laquelle les termes A, R₄, R₅ et R₆ ont la même signification que précédemment.

Ces réactions de silylation sont effectuées dans les conditions connues dans l'art antérieur.

Les dérivés de l'aminoacide ou du peptide sont introduits avec l'agent silylant dans un solvant tel que par exemple un éther (tétrahydrofuranne), un solvant aliphatique halogéné, un ester, un nitrile (acétonitrile) ou un amide (DMF).

Selon une méthode de mise en oeuvre on utilise notamment des concentrations en aminoacide ou peptide dans le solvant comprise entre 0,1 et 1 mole par litre.

On utilise avantageusement des concentrations en agent silylant par rapport à l'aminoacide ou au peptide comprises entre 1 et 3 moles.

Pour une meilleure mise en oeuvre de l'invention il est préférable au cours de la deuxième étape ou étape d'activation qui consiste à mettre en contact un peptide ou un amino-acide dont la fonction amine est protégée et la fonction acide est O-silylée avec un sel complexe de chloroimmonium de formule (IV), d'halogénure de phosphore de formule (V), d'oxyhalogénure de phospore ou d'halogénure d'oxyalyle, d'opérer sous un courant de gaz inerte tel que l'azote ou l'argon, dans un solvant aliphatique ou aromatique halogéné. On peut utiliser tout solvant permettant de solubiliser le peptide ou l'aminoacide activé et ne réagissant ni avec lui ni avec les sels complexes. Le solvant est choisi notamment parmi le chlorure de méthylène, le chloroforme, le dichloro-1,2 éthane, le chlorobenzène.

On préfère utiliser une quantité de sel complexe telle que le rapport molaire du sel complexe de chloroimmonium, du sel complexe d'halogénure ou d'oxyhalogénure de phosphore ou du sel complexe d'halogénure d'oxalyle aux peptides ou à l'aminoacide soit compris entre 1,1 et 1,3.

La réaction entre le sel complexe de chloroimmonium, d'halogénure de phosphore, d'oxyhalogénure de phosphore ou d'halogénure d'oxalyle et l'aminoacide ou le peptide est réalisée de préférence à une température comprise entre - 20 et 30°C. On utilise de préférence une concentration molaire en sel complexe, en aminoacide ou en peptide dans le solvant compris entre 0,01 et 0,1 mole/litre.

La durée de l'étape d'activation varie avantageusement entre 30 minutes et 2 heures.

En ce qui concerne la réaction de couplage (C) il est connu dans l'art antérieur de coupler des dérivés silylés sur la fonction carboxyle et sur la fonction aminée mais dans aucun de ces procédés n'est décrit le couplage entre un dérivé activé par un groupe immonium ou par les réactifs indiqués ci-dessus et un dérivé silylé sur la fonction azotée. Or ce couplage bien précis apporte des résultats quant à l'absence de racémisation tout à fait inattendus.

La réaction de couplage ou troisième étape du procédé de l'invention est réalisée par addition de préférence du composé aminé sylilé à la solution du peptide ou de l'aminoacide activé sous atmosphère de gaz inerte et de préférence à une température comprise entre - 10 et 30°C. On utilise avantageusement lorsque l'on utilise un sel complexe d'antimoine un additif choisi parmi les phosphites d'alkyle. On préfère utiliser le phosphite de méthyle. On ajoute avantageusement une quantité de phosphite telle que le rapport molaire par rapport à l'aminoacide activé ou au peptide activé soit compris entre 2 et 3.

Avantageusement avec les sels d'halogénure et d'oxyhalogénure de phosphore ou d'halogénure d'oxalyle on peut procéder simultanément à l'étape d'activation et de couplage.

La durée de l'étape de couplage varie préférentiellement entre 5 et 24 heures.

L'invention sera plus complètement décrite à l'aide des exemples suivants qui ne doivent en aucun cas être considérés comme limitatifs de l'invention.

### Exemple 1

Dans un ballon tricol muni d'un thermomètre, d'un agitateur et d'une ampoule à brome et maintenu sous azote, on place une solution de 1 ml (10 meq) de trichlorure de phosphore dans 8 ml de chloroforme. On refroidit à - 20°C et ajoute goutte à goutte avec agitation 2 ml (13 meq) de pentachlorure d'antimoine dans 8 ml de chloroforme. L'addition terminée, on agite encore 10 min à - 20°C, puis laisse revenir à la température ambiante durant 1 h. On refroidit à - 20°C, le précipité est filtré, et lavé 3 fois avec de petites quantités de chloroforme froid. On sèche finalement sous vide sans chauffer. Le sel complexe de formule (V) dans lequel M = Sb, n = 5, et X = Cl fond à 105-107° (Rendement 90 %).

### Exemple 2

0,732 g (2,5 meq) d'ester triméthylsilylique de la benzoyl-L-valine, 0,51 g (2,5 meq) d'ester méthylique de la N-triméthylsilyl L-valine et 1 ml (7,5 meq) de phosphite de méthyle sont dissous dans 50 ml de chlorure de méthylène. On ajoute sous azote 1,31 g (3 meq) de sel complexe obtenu à l'exemple 1 et laisse agiter 12 h à la température ambiante. On filtre, lave trois fois avec de l'acide citrique N, trois fois avec une solution saturée de bicarbonate de sodium, une fois à l'eau, sèche sur sulfate de magnésium et chasse le solvant sous vide.

Le dipeptide Bz-Val-Gly-OCH₃ qui sert de test pour la détermination du rendement en chromatographie liquide haute performance est introduit avant les lavages.

Le rendement en Bz-Val-Val-OCH₃ et le taux de racémisation (exprimé en DL %) sont déterminés à partir du résidu brut par chromatographie liquide haute performance en phase inverse, sur colonne C 18 Ultrashere Altex, avec comme éluant un mélange CH₃OH 52 % - H₂O 48 % et un débit de 1 ml/min (détection à 254 nm).
Rendement 65 % DL % = 0 %.

### Exemple 3

On opère comme dans l'Exemple 2, mais en utilisant l'ester méthylique de la N-triméthylsilyl L-proline comme composant aminé.

Le rendement en Bz-Val-Pro-OCH₃ et le taux de racémisation sont déterminés par chromatographie liquide haute performance en phase inverse, sur colonne C 18 Ultrasphere Altex, avec comme éluant un mélange CH₃OH 48 % - H₂O 52 % et un débit de 1 ml/min.
Rendement 55 % DL % = 1 %.

### Exemple 4

A 1,02 g (5 meq) d'ester méthylique de la N-triméthylsilyl L-valine dans 20 ml de chlorure de méthylène, on ajoute sous azote successivement 0,42 ml (5 meq) de trichlorure de phosphore et 0,952 g (7 meq) de chlorure de zinc sec. Après 2 h à 20°, on additionne 0,732 g (2,5 meq) d'ester triméthylsylylique de la benzoyl-L-valine dissous dans 20 ml de chlorure de méthylène. On agite finalement sous azote 12 h à 20°, et traite comme indiqué dans l'exemple 2.
Rendement 67 % DL = 0 %.

### Exemple 5

0,557 g (2,5 meq) de l'ester β-benzylique de l'acide N-t-butyloxycarbonyl L-aspartique et un excès d'hexaméthyldisilazane dans 20 ml de chlorure de méthylène anhydre sont agités sous azote 2 h à la température ambiante. On concentre sous vide sans chauffer et reprend le résidu par 50 ml de CH₂Cl₂. On ajoute alors 0,63 g (2,5 meq) d'ester méthylique de la N-triméthylsilyl L-phénylalanine, 0,476 g (3,5 meq) de chlorure de zinc sec, 0,343 g (2,5 meq) de trichlorure de phosphore et agite 12 h à la température ambiante sous azote. On filtre, lave trois fois avec de l'acide citrique N, trois fois avec une solution saturée de bicarbonate de sodium, une fois à l'eau, sèche sur sulfate de magnésium et chasse le solvant sous vide.

Le rendement en Boc-Asp (OBzl)-Phe-OCH₃ est de 65 %. Huile caractérisée par son spectre RMN et par son spectre de masse.

Le déprotection en Aspartame est finalement réalisée selon les méthodes de l'art. On réduit catalytiquement pour éliminer le reste benzyle de l'acide aspartique et on traite par l'acide trifluoracétique pour éliminer le groupe Boc.

### Exemple 6

Préparation de l'hexachloroantimoniate de diméthylchloroimmonium (IV) (Arnold et Holy, Coll. Czech. Chem. Comm., 1962, 27, 2886).

Une solution de 2,97 g de pentachlorure d'antimoine dans 10 ml de chloroforme est ajoutée goutte avec agitation à - 40°C à 5 ml d'une solution 2 M de chlorure de diméthylchloroimmonium dans le chloroforme. Le précipité est filtré, lavé 2 fois avec du chloroforme et séché. F. 162-164°C (Rendement 90 %).

### Exemple 7

1,28 g (3 meq) d'hexachloroantimoniate de diméthylchloroimmonium (IV) (R = R′ = CH₃, R˝ = H, M = Sb, n = 5, X = Cl) sont mis en suspension sous azote dans 50 ml de chlorure de méthylène refroidi à - 20°C. On ajoute goutte à goutte avec agitation 0,732 g (2,5 meq) d'ester triméthylsilylique de la benzoyl-L-valine dissous dans 5 ml de CH₂Cl₂. Après dissolution du réactif, on agite encore 30 min à - 20°C. On ajoute alors 0,93 g (7,5 meq) de phosphite de méthyle ; après 15 min d'agitation à - 20°C, on introduit toujours sous azote 0,51 g (2,5 meq) d'ester méthylique de la N-triméthylsilyl-L-valine. La mélange réactionnel est agité 1 h à - 20°C, puis on laisse lentement revenir à la température ambiante et agite 12 h à 20°C. On filtre, lave le filtrat 3 fois avec de l'acide citrique N, 2 fois avec une solution saturée de bicarbonate de sodium et une fois à l'eau. On sèche sur sulfate de magnésium et chasse le solvant sous vide.
Rendement 50 % DL % 0,2.

### Exemple 7 comparatif

On opère comme dans l'exemple 7, mais en l'absence de phosphite de méthyle. Le rendement est d'environ 20 % ; la détermination de la DL est impossible avec précision, le rendement étant trop faible.

### Exemple 8

Un mélange de 0,21 ml (2,5 meq) de chlorure d'oxalyle et de 0,44 ml (3,5 meq) de pentachlorure d'antimoine dilué dans 2 ml de chlorure de méthylène est ajouté sous azote goutte à goutte avec agitation à 0° à une solution de 0,732 g (2,5 meq) de benzoyl-L-valine dans 50 ml de chlorure de méthylène. L'agitation est maintenue 1 h à 20°. On ajoute alors 0,975 g (7,5 meq) de phosphite de méthyle, puis après 5 min d'agitation, 0,51 g (2,5 meq) d'ester méthylique de la N-triméthylsilyl L-valine. On agite finalement 12 h à 20°, toujours sous azote. Le traitement est identique à celui indiqué dans l'exemple 2.
Rendement 51 % DL 0%

### Exemple 9

0,44 g (5 meq) de diméthylformamide et 0,78 g (3,5 meq) de bromure de zinc sec sont mis en solution dans 30 ml de chlorure de méthylène. On refroidit à -20° et ajoute goutte à goutte sous azote 0,25 ml (3 meq) de chlorure d'oxalyle dilué dans 5ml de CH₂Cl₂. Après 30 minutes d'agitation à -20°, on ajoute goutte à goutte avec agitation 0,732 g (2,5 meq) d'ester triméthylsilylique de la benzoyl-L-valine en solution dans 15 ml de chlorure de méthylène. On agite 40 minutes en laissant la température revenir lentement jusqu'à 0°. On ajoute alors 0,51 g (2,5 meq) d'ester méthylique de la N-triméthylsilyl L-valine, on laisse agiter sous azote 12 heures à la température ambiante et traite comme indiqué dans l'exemple 2.
Rendement 95 - 100% DL 0%

## Revendications

1. Procédé de préparation de synthons peptidiques optiquement purs caractérisé en ce que dans une première étape on prépare un dérivé O-silylé d'un peptide d'un aminoacide dont la fonction azotée est protégée,
dans une deuxième étape on active le peptide ou l'aminoacide O-silylé par un sel complexe de chloroimmonium (c'est-à-dire un sel comportant un anion complexe de formule MX⁻ₙ₊₁) ou un sel complexe de dichlorophosphénium,
dans une troisième étape on condense le peptide ou l'aminoacide activé sur un peptide ou un aminoacide dont la fonction amine a été N-sylilée.

2. Procédé selon la revendication 1 caractérisé en ce que le peptide de la première étape contient plus de 2 aminoacides.

3. Procédé selon la revendication 1 caractérisé en ce que on opère au cours des trois étapes en l'absence de dérivés basiques.

4. Procédé selon la revendication 1 caractérisé en ce que le sel complexe de chloroimmonium répond à la formule (IV) : 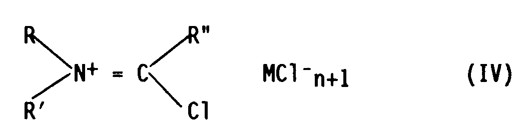 dans laquelle :
* R et R', identiques ou différents, représentent un radical alkyle ayant de préférence de 1 à 5 atomes de carbone, ou encore un radical divalent de formule
-(CH₂)ₘ(X)_{m'} - (CH₂)ₘ-
dans laquelle :
. X est un groupe méthylène ou un hétéroatome tel que l'atome d'oxygène ou d'azote,
. m est un nombre entier de 2 à 6, de préférence égal à 2,
. m′ est un nombre entier égal à 0 ou 1,
* R'' est un atome d'hydrogène ou un groupement alkyle, alkényle, aryle ou aralkyle.
* M représente un métal choisi parmi le zinc, l'aluminium, l'antimoine, le titane et l'étain.
* n est un nombre entier égal ou supérieur à 2 et égal ou inférieur à 5.

5. Procédé selon la revendication 1 caractérisé en ce que le sel complexe d'halogénure de phosphore répond à la formule générale (V) : 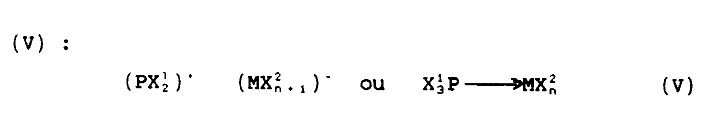 dans laquelle
. M représente un atome d'antimoine et n = 5 ou un atome de zinc et n = 2.
. X¹ et X² représentent un halogène identique ou différent choisi parmi le chlore, le brome et l'iode.

6. Procédé selon la revendication 1 caractérisé en ce que l'on utilise un sel complexe d'oxyhalogénure de phosphore ou d'halogénure d'oxalyle et de chlorure de zinc ou de pentachlorure d'antimoine.

7. Procédé selon la revendication 1 caractérisé en ce que on utilise une quantité de sel complexe de chloroimmonium, de trichlorure de phosphore ou de chlorure d'oxalyle telle que le rapport molaire dudit sel au peptide ou à l'aminoacide est compris entre 1,1 et 1,3.

8. Procédé selon la revendication 1 caractérisé en ce que la toutes les étapes sont réalisées sous courant de gaz inerte, dans une solvant aliphatique ou aromatique halogéné.

9. Procédé selon la revendication 8 caractérisé en ce que le solvant est choisi parmi le chlorure de méthylène, le chloroforme, le dichloro-1,2 éthane, le chlorobenzène.

10. Procédé selon les revendications 1 et 7 à 9 caractérisé en ce que la deuxième étape est réalisée de préférence à une température comprise entre - 20 et 30°C.

11. Procédé selon la revendication 1 caractérisé en ce que lors de la deuxième étape on utilise une concentration molaire en aminoacide activé, en peptide activé dans le solvant comprise entre 0,01 et 0,1 mole par litre.

12. Procédé selon la revendication 1 caractérisé en ce que au cours de la troisième étape lorsque l'on utilise les sels complexes à base d'antimoine on ajoute un additif choisi parmi les phosphites d'alkyle.

13. Sels complexes de phosphore de formule (V) 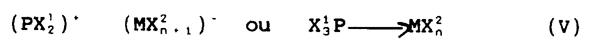 dans laquelle
. X¹ et X² représentent un halogène choisi parmi le chlore, le brome ou l'iode
. M représente un métal choisi parmi l'antimoine et le zinc.
. n est égal à 5 lorsque M représente l'antimoine et n = 2 lorsque M représente le zinc.

## Claims

1. Process for preparing optically pure peptide synthons, characterised in that, in a first stage, an O-silyl derivative of a peptide of an amino acid, in which the nitrogenous group is protected, is prepared,
in a second stage, the O-silyl peptide or amino acid is activated by a complex chloroimmonium salt, (that is to say a salt containing a complex anion of formula MX^{⁻}ₙ₊₁) or a complex dichlorophosphenium salt,
in a third stage, the activated peptide or amino acid is condensed with a peptide or amino acid in which the amine group has been N-silylated.

2. Process according to Claim 1, characterised in that the peptide of the first stage contains more than 2 amino acids.

3. Process according to Claim 1, characterised in that the three stages are performed in the absence of basic derivatives.

4. Process according to Claim 1, characterised in that the complex chloroimmonium salt corresponds to the formula (IV): 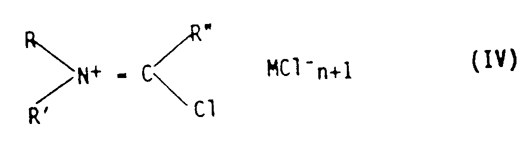 in which:
* R and R', which may be identical or different, denote an alkyl radical preferably having from 1 to 5 carbon atoms, or alternatively a divalent radical of formula
-(CH₂)ₘ-(X)_{m'} - (CH₂)m-
in which
· X is a methylene group or a hetero atom such as an oxygen or nitrogen atom,
· m is an integer from 2 to 6, and preferably equal to 2,
· m' is an integer equal to 0 or 1;
* R'' is a hydrogen atom or an alxyl, alkenyl, aryl or aralkyl group;
* M denotes a metal chosen from zinc, aluminium, antimony, titanium and tin;
* n is an integer equal to or greater than 2 and equal to or less than 5.

5. Process according to Claim 1, characterised in that the complex phosphorus halide salt corresponds to the general formula (V): 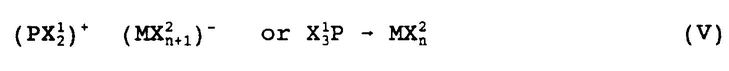 in which
· M denotes an antimony atom and n = 5, or a zinc atom and n = 2;
· X¹ and X² denote an identical or different halogen chosen from chlorine, bromine and iodine.

6. Process according to Claim 1, characterised in that a complex salt of phosphorus oxyhalide or oxalyl halide and of zinc chloride or antimony pentachloride is used.

7. Process according to Claim 1, characterised in that a quantity of complex chloroimmonium, phosphorus trichloride or oxalyl chloride salt is used such that the mole ratio of the said salt to the peptide or the amino acid is between 1.1 and 1.3.

8. Process according to Claim 1, characterised in that all the stages are carried out under a stream of inert gas, in a halogenated aromatic or aliphatic solvent.

9. Process according to Claim 8, characterised in that the solvent is chosen from methylene chloride, chloroform, 1,2-dichloroethane and chlorobenzene.

10. Process according to Claims 1 and 7 to 9, characterised in that the second stage is preferably carried out at a temperature of between -20 and 30°C.

11. Process according to Claim 1, characterised in that, during the second stage, a molar concentration of activated amino acid or activated peptide in the solvent of between 0.01 and 0.1 mole per litre is used.

12. Process according to Claim 1, characterised in that, during the third stage, when complex salts based on antimony are used, an additive chosen from alkyl phosphites is added.

13. Complex phosphorus salts of formula (V) 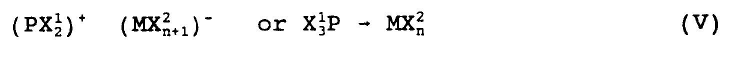 in which
· X¹ and X² denote halogen chosen from chlorine, bromine or iodine;
· M denotes a metal chosen from antimony and zinc;
· n is equal to 5 when M denotes antimony, and n = 2 when M denotes zinc.

## Patentansprüche

1. Verfahren zur Herstellung optisch reiner Peptid-synthons dadurch gekennzeichnet, daß man in einer ersten Stufe ein O-silyliertes Derivat einer Aminosäure eines Peptids herstellt, dessen Stickstofffunktion geschützt ist,
man in einer zweiten Stufe das Peptid oder die O-silylierte Aminosäure durch ein komplexes Chlorimmoniumsalz aktiviert (das heißt ein Salz, das ein komplexes Anion der Formel MX^{⁻}ₙ₊₁ enthält) oder durch ein komplexes Dichlorphosphorsalz,
man in einer dritten Stufe das Peptid oder die aktivierte Aminosäure an ein Peptid oder eine Aminosäure koppelt, deren Aminofunktion N-silyliert wurde.

2. Verfahren nach Anspruch 1, dadurch gekennzeichnet, daß das Peptid aus der ersten Stufe mehr als zwei Aminosäuren enthält.

3. Verfahren nach Anspruch 1, dadurch gekennzeichnet, daß man im Lauf der drei Stufen in der Abwesenheit basischer Derivate arbeitet.

4. Verfahren nach Anspruch 1, dadurch gekennzeichnet, daß das komplexe Chlorimmoniumsalz der Formel (IV) entspricht: 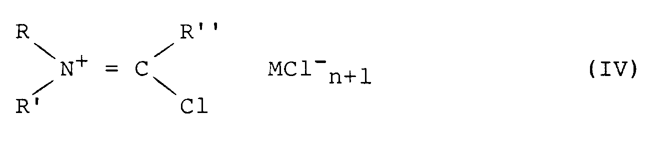 in der:
* R und R', identisch oder verschieden, für einen Alkylrest stehen, der bevorzugt zwischen eins und fünf Kohlenstoffatome hat, oder auch für einen zweiwertigen Rest der Formel
-(CH₂)ₘ-(X)_{m'} - (CH₂)ₘ-
in der:
· X eine Methylengruppe ist oder ein Heteroatom wie Sauerstoff oder Stickstoff,
· m eine ganze Zahl von zwei bis sechs ist, bevorzugt gleich zwei,
· m' eine ganze Zahl gleich null oder eins ist,
* R'' ein Wasserstoffatom oder eine Alkyl-, Alkenyl-, Aryl- oder eine Aralkylgruppe ist,
* M steht für ein Metall wie Zink, Aluminium, Antimon, Titan und Zinn,
* n ist eine ganze Zahl gleich oder größer als zwei und kleiner oder gleich fünf.

5. Verfahren nach Anspruch 1, dadurch gekennzeichnet, daß das komplexe Halogenphosphorsalz der allgemeinen Formel (V) entspricht: 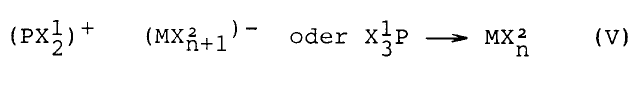 in der
· M für ein Antimonatom und n = 5 steht oder für ein Zinkatom und n = 2,
· X¹ und X² für ein Halogen stehen, identisch oder verschieden, wobei das Halogen Chlor, Brom und Jod sein kann.

6. Verfahren nach Anspruch 1, dadurch gekennzeichnet, daß man ein komplexes Salz des oxyhalogenierten Phosphors oder Oxalylhalogens und Zinkchlorids oder Antimonpentachlorid verwendet.

7. Verfahren nach Anspruch 1, dadurch gekennzeichnet, daß man eine Menge an komplexen Chlorimmoniumsalz, Phosphortrichlorid oder Oxalylchlorid verwendet, derart, daß das molare Verhältnis des besagten Salzes zum Peptid oder zur Aminosäure zwischen 1,1 und 1,3 liegt.

8. Verfahren nach Anspruch 1, dadurch gekennzeichnet, daß alle Stufen unter Inertgasfluß ablaufen, in einem aliphatischen oder halogeniert aromatischen Lösungsmittel.

9. Verfahren nach Anspruch 8, dadurch gekennzeichnet, daß das Lösungsmittel aus Methylenchlorid, Chloroform, 1,2-Dichlorethan oder Chlorbenzol ausgewählt wird.

10. Verfahren nach den Ansprüche 1 und 7 bis 9, dadurch gekennzeichnet, daß die zweite Stufe bevorzugt bei einer Temperatur zwischen -20 und 30°C durchgeführt wird.

11. Verfahren nach Anspruch 1, dadurch gekennzeichnet, daß man während der zweiten Stufe eine molare Konzentration der aktivierten Aminosäure oder des aktivierten Peptids im Lösungsmittel verwendet, die zwischen 0,01 und 0,1 Mol pro Liter liegt.

12. Verfahren nach Anspruch 1, dadurch gekennzeichnet, daß man im Lauf der dritten Stufe, wenn man die Komplexsalze auf Antimonbasis verwendet, einen Zusatz hinzufügt, der aus den Alkylphosphiten ausgewählt wird.

13. Komplexsalze des Phosphors der Formel (V) 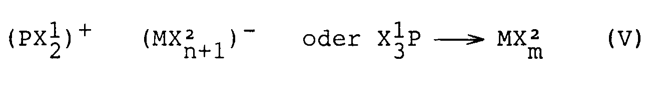 in der
· X¹ und X² für ein Halogen stehen, wobei das Halogen für Chlor, Brom und Jod stehen kann,
· M für ein Metall wie Antimon oder Zink steht,
· n gleich fünf ist, wenn M für Antimon steht und n = 2 ist, wenn M für Zink steht.
